# EUROPEAN PATENT APPLICATION

(11) **EP 2 033 689 A1**
(43) Date of publication of application: **11.03.2009**
(21) Application number: 07016441.3
(22) Date of filing: 22.08.2007
(51) Int. Cl.: A61Q 19/08, A61K 8/46, A61K 8/67, A61K 8/73

(54) **Injectable dermatological composition for treatment of the wrinkles**

(71) Applicant: Italfarmacia S.r.l., 00155 Roma (IT)
(72) Inventor: Ceccarelli, Maurizio, 00123 Roma (IT); Piccioli, Piero, 00162 Roma (IT); Marchetti, Mario, 00161 Roma (IT); Cavallo, Giovanni, 00122 Ostia (Roma) (IT)
(74) Representative: Celestino, Marco

(57) **Abstract**

Injectable dermatological composition for treatment of skin wrinkles comprising at least one aminoacid adapted to promote the synthesis of collagen and at least one sulphurated aminoacid, The sulphurated aminoacid can be selected from the group comprised of: cysteine SH, methionine SH and glutathione, or a combination thereof. In particular, the or each sulphurated aminoacid inhibits the activity of metalloproteinase and extends the duration of the effects of hyaluronic acid. The injectable dermatological composition has a reduced possibility of having undesirable side effects. The injectable dermatological composition for the treatment of skin wrinkles also comprises at least one metalloproteinase inhibitor capable of extending the duration of the anti-wrinkle effects and assisting the inhibitory action of the sulphurated aminoacid with respect to the metalloproteinase.

## Description

### Field of the invention.

The present invention relates to an injectable dermatological composition for treating lesions of the skin and, in particular, for topical treatment of wrinkles.

### Description of the prior art.

As well known, skin is an continuously changing tissue comprising a plurality of cells and specialized structures. Being continuously into contact with the environment, the skin represents a protective barrier for the body. It is, furthermore, involved in main processes that allow to the body of maintaining a fixed temperature.

Furthermore, the skin plays a relevant role in the immune system that protects the body from diseases.

In particular, the skin comprises three layers, i.e. an external layer, the epidermis, an inner layer called subcutaneous tissue, and an intermediate layer, commonly called dermis.

The epidermis in quality of outer layer acts as barrier against the external agents and has a different thickness according to the anatomic part that it covers. For example, the thickness of the epidermis at the eyelids is about 0.05 mm, whereas the thickness of the epidermis of the foot sole and of the palm of the hand is about 1.5 mm.

The epidermis in turn has 5 layers, i.e. the stratum basale, the stratum spinosum, the stratum germinativum, the pigment layer and the stratum corneum.

Like epidermis, also dermis has different thicknesses according to its location in the body skin, changing from about 0.3 mm at the eyelids to 3.0 mm of the backside. Dermis has three different tissues, i.e. collagen tissue, resilient tissue and reticular fibres.

The subcutaneous tissue, finally, is a layer of adipose tissue and connective tissue and covers the nerves and the larger blood vessels. The size of this tissue varies personally and depending on the zone of the body.

The wrinkles and thin lines are visible signs of the everyday obstacles of the skin. In particular, a softening of the tissues assists the production of slits of different depths. The slits eventually come to the dermis, which works as support to epidermis and is responsible of tonicity of the skin. In particular, when the dermis looses its elasticity, it weakens and begin to form deeper wrinkles. During the production of wrinkles the fibers of collagen, responsible of the elasticity and of the structure of the skin, loose their characteristics with an overproduction of enzymes of metalloproteinase type. These anomalous amount of enzymes degrade the matrix of collagen up to the production of deep wrinkles.

Other factors, such as free radicals, the exposition to sun, pollution, smoke, ozone, can damage the skin. In particular, the free radicals with oxygen bring to the production of wrinkles through the activation of metalloproteinase enzymes that cause the decomposition of collagen.

During the years the dermis tends to become thinner, in particular, at the collagen layer. At the same time the atrophy of adipose cells, localized under the dermis proceeds continuously. Therefore, the skin becomes dry and scaly.

An attempt to stop, or at least to decelerate, the above described processes that cause in different ways the production of wrinkles, different approaches have been followed, and in particular: by supplying stimulants of the synthesis of collagen, by subcutaneous injections of natural polymers, by using antiradical/antioxidant agents.

However, the above indicated treatments are not completely effective and are not capable of assuring long lasting results.

For example, among the treatments that provide injections of natural polymers for reducing the wrinkles, the most common provides hyaluronic acid. Hyaluronic acid, which is normally present in the dermis, can be injected as a filler that can be reabsorbed. Immediately after, or within a few hours, from the injection, the site of operation can redden and swell for then returning to normality within one week. This type of treatment requires at least two injections for achieving perceivable effects. In fact, hyaluronic acid is not permanent and eventually degrades and is reabsorbed by the body. Therefore, it is necessary to repeat the injections periodically.

Other treatments provide the supply of ascorbic acid, or vitamin C, essential for an effective synthesis of collagen. However, notwithstanding vitamin C is useful for reconstructing collagen of the skin and for reducing the wrinkles, may be not beneficial and may have noxious effects if used improperly.

### Summary of the invention

It is therefore a feature of the present invention to provide an injectable dermatological composition for treatment of skin wrinkles that is capable of assuring a long lasting effect.

It is also a feature of the present invention to provide an injectable dermatological composition for treatment of skin wrinkles that is capable of reducing undesirable side effects.

These and other features are accomplished with one exemplary injectable dermatological composition for treatment of skin wrinkles, according to the invention, comprising:
- at least one aminoacid adapted to promote the synthesis of collagen;
- at least one sulphurated aminoacid.

In particular, the sulphurated aminoacid can be selected from the group comprised of:
- cysteine SH;
- methionine SH;
- glutathione,
or a combination thereof.

In particular, the or each sulphurated aminoacid inhibits the activity of metalloproteinase and capable of extending the duration of effects of hyaluronic acid.

Advantageously, the injectable dermatological composition for treatment of skin wrinkles comprises also at least one metalloproteinase inhibitor capable of extending the duration of effects and assist the inhibitory action of the or each sulphurated acid with respect to the metalloproteinase.

In particular, the metalloproteinase inhibitor can be selected from the group comprised of:
- lipoic acid;
- EDTA (ethylenediaminetetraacetate);
- EDTA salts;
or a combination thereof.

In particular, the injectable dermatological composition for treatment of skin wrinkles above described can provide, furthermore, at least one propmoter of collagen synthesis, such as magnesium ascorbyl phosphate.

Advantageously, the aminoacid adapted to promote the synthesis of collagen can be selected from the group comprised of:
- L-isoleucine;
- L-leucine;
- L-lysine HCL;
- L-proline;
- L-valine;
- L-glycine;
- L-serine;
- L-alanine;
or a combination thereof.

Advantageously, the injectable dermatological composition comprises also a natural polymer, selected from the group comprised of:
- collagen;
- hyaluronic acid;
- Dextran.

In particular, fractions of Dextran are used having a molecular weight set between 1.000 and 2.000.000 Daltons, advantageously set between 40.000 and 2.000.000 Daltons.

Advantageously, to assist the synthesis of collagen the dermatological composition above described comprises also a derivative of ascorbic acid, i.e. magnesium ascorbyl phosphate.

The injectable dermatological composition can advantageously comprise also a determined amount of antioxidant, such as glutathione and vitamin E.

A possible injectable dermatological composition according to the invention comprises
- A mixture based on aminoacids adapted to promote the synthesis of collagen and sulphurated aminoacids (da 0,01 g - 5 gr);
- acid ascorbic or its derivatives (0,01 g - 2 gr);
- acid Lipoic (0,01 - 1 gr);
- EDTA or thereof salt (0,001 - 1 gr);
- hyaluronic acid or thereof salt (0,001 - 2 gr);
- Dextran (0,001 - 2 gr).

Advantageously, an injectable dermatological composition, according to the invention, comprises:
- L-isoleucine;
- L-leucine;
- L-lysine HCL;
- L-proline;
- L-valine;
- L-glycine;
- L-serine;
- L-alanine;
- Cysteine SH;
- glutathione GSH;
- Methionine SH.

An injectable dermatological composition alternative for treatment of skin wrinkles comprises:
- L-isoleucine;
- L-leucine;
- L-lysine HCL;
- L-proline;
- L-valine;
- L-glycine;
- L-serine;
- L-alanine;
- Cysteine SH;
- Methionine SH;
- Magnesium ascorbyl phosphate;
- Lipoic acid;
- EDTA.

A further exemplary embodiment of the injectable dermatological composition for treatment of skin wrinkles comprises:
- L-isoleucine;
- L-leucine;
- L-lysine HCL;
- L-proline;
- L-valine;
- L-glycine;
- L-serine;
- L-alanine;
- Cysteine SH;
- Methionine SH;
- Magnesium ascorbyl phosphate;
- Lipoic acid;
- EDTA(ethylenediaminetetraacetate);
- EDTA sodium salt;
- Hyaluronic acid;
- Dextran.

In the following tables some examples are given of dermatological compositions, according to the present invention, for treatment of skin wrinkles.

### EXAMPLE 1

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Magnesium ascorbyl phosphate | 0.20 |
| EDTA | 0.05 |
| Sodium carbonate | 1.80 |
| Water | up to 100 g |

### EXAMPLE 2

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Magnesium ascorbyl phosphate | 0.20 |
| EDTA | 0.05 |
| Sodium carbonate | 1.80 |
| Water | up to 100 g |

### EXAMPLE 3

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Magnesium ascorbyl phosphate | 0.02 |
| EDTA | 0.05 |
| Sodium carbonate | 1.80 |
| Lipoic acid | |
| Water | up to 100 g |

### EXAMPLE 4

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Magnesium ascorbyl phosphate | 0.20 |
| EDTA | 0.05 |
| Buffer of phosphate | ---- |
| Water | up to 100 g |

### EXAMPLE 5

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Magnesium ascorbyl phosphate | 0.20 |
| EDTA | 0.05 |
| Buffer of phosphate | ---- |
| Water | up to 100 g |

### EXAMPLE 6

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Magnesium ascorbyl phosphate | 0.20 |
| Lipoic acid | 0.10 |
| EDTA | 0.05 |
| Buffer of phosphate | ---- |
| Water | up to 100 g |

### EXAMPLE 7

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Magnesium ascorbyl phosphate | 0.20 |
| EDTA | 0.05 |
| Sodium chloride | ---- |
| Water | up to 100 g |

### EXAMPLE 8

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Magnesium ascorbyl phosphate | 0.20 |
| EDTA | 0.05 |
| Sodium chloride | ---- |
| Water | up to 100 g |

### EXAMPLE 9

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Magnesium ascorbyl phosphate | 0.20 |
| EDTA | 0.05 |
| Sodium chloride | ---- |
| Water | up to 100 g |

### EXAMPLE 10

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Sodium chloride | ---- |
| Water | up to 100 g |

### EXAMPLE 11

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Sodium carbonate | 1.80 |
| Water | up to 100 g |

### EXAMPLE 12

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Dextran (40.000 - 2.000.000) | 0.10 |
| Sodium carbonate | 1.80 |
| Water | up to 100 g |

### EXAMPLE 13

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| magnesium ascorbyl phosphate | 0.20 |
| EDTA | 0.05 |
| Hyaluronic acid sodium salt | 0.10 |
| Dextran (40.000 - 2.000.000) | 0.10 |
| Glucosamine sulphate | 0.08 |
| Sodium carbonate | 1.80 |
| Water | up to 100 g |

### EXAMPLE 14

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Glucosamine sulphate | 0.08 |
| Sodium carbonate | 1.80 |
| Water | up to 100 g |

### EXAMPLE 15

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Glucosamine sulphate | 0.08 |
| Dextran (40.000 - 2.000.000) | 0.05 |
| Sodium carbonate | 1.80 |
| Water | up to 100 g |

### EXAMPLE 16

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Phosphate buffer | --- |
| Sodium carbonate | 1.80 |
| Water | up to 100 g |

### EXAMPLE 17

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Dextran (40.000 - 2.000.000) | 0.10 |
| Phosphate buffer | --- |
| Sodium carbonate | 1.80 |
| Water | up to 100 g |

### EXAMPLE 18

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Dextran (40.000 - 2.000.000) | 0.10 |
| Glucosamine sulphate | 0.08 |
| Phosphate buffer | --- |
| Water | up to 100 g |

### EXAMPLE 19

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Glucosamine sulphate | 0.08 |
| Phosphate buffer | --- |
| Water | up to 100 g |

### EXAMPLE 20

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| magnesium ascorbyl phosphate | 0.20 |
| EDTA | 0.05 |
| Hyaluronic acid sodium salt | 0.10 |
| Glucosamine sulphate | 0.08 |
| Dextran (40.000 - 2.000.000) | 0.05 |
| Phosphate buffer | --- |
| Water | up to 100 g |

### EXAMPLE 21

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Phosphate buffer | --- |
| Water | up to 100 g |

### EXAMPLE 22

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Dextran (40.000 - 2.000.000) | 0.05 |
| Phosphate buffer | --- |
| Water | up to 100 g |

### EXAMPLE 23

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Glucosamine sulphate | 0.08 |
| Dextran (40.000 - 2.000.000) | 0.10 |
| Phosphate buffer | --- |
| Water | up to 100 g |

### EXAMPLE 24

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| magnesium ascorbyl phosphate | 0.20 |
| EDTA | 0.05 |
| Hyaluronic acid sodium salt | 0.10 |
| Glucosamine sulphate | 0.08 |
| Phosphate buffer | --- |
| Water | up to 100 g |

### EXAMPLE 25

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| magnesium ascorbyl phosphate | 0.20 |
| EDTA | 0.05 |
| Hyaluronic acid sodium salt | 0.10 |
| Glucosamine sulphate | 0.08 |
| Dextran (40.000 - 2.000.000) | 0.05 |
| Phosphate buffer | --- |
| Water | up to 100 g |

### EXAMPLE 26

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Dextran (40.000 - 2.000.000) | 0.05 |
| L-glutathione | 0.01 |
| Vitamin E acetate | 0.01 |
| Sodium carbonate | 1.80 |
| Water | up to 100 g |

### EXAMPLE 27

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Dextran (40.000 - 2.000.000) | 0.05 |
| Glucosamine sulphate | 0.08 |
| Vitamin E acetate | 0.01 |
| Sodium carbonate | 1.80 |
| Water | up to 100 g |

### EXAMPLE 28

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Dextran (40.000 - 2.000.000) | 0.05 |
| Glucosamine sulphate | 0.08 |
| L-glutathione | 0.01 |
| Vitamin E acetate | 0.01 |
| Sodium carbonate | 1.80 |
| Water | up to 100 g |

### EXAMPLE 29

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Dextran (40.000 - 2.000.000) | 0.05 |
| Vitamin E acetate | 0.01 |
| Phosphate buffer | --- |
| Water | up to 100 g |

### EXAMPLE 30

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Dextran (40.000 - 2.000.000) | 0.05 |
| L-glutathione | 0.01 |
| Vitamin E acetate | 0.01 |
| Phosphate buffer | --- |
| Water | up to 100 g |

### EXAMPLE 31

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Dextran (40.000 - 2.000.000) | 0.05 |
| Glucosamine sulphate | 0.08 |
| L-glutathione | 0.01 |
| Vitamin E acetate | 0.01 |
| Phosphate buffer | --- |
| Water | up to 100 g |

### EXAMPLE 32

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Dextran (40.000 - 2.000.000) | 0.05 |
| Glucosamine sulphate | 0.08 |
| L-glutathione | 0.01 |
| Vitamin E acetate | 0.01 |
| Phosphate buffer | --- |
| Water | up to 100 g |

### EXAMPLE 33

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| Dextran (40.000 - 2.000.000) | 0.05 |
| Sodium chloride | ---- |
| Vitamin E acetate | 0.01 |
| Phosphate buffer | --- |
| Water | up to 100 g |

### EXAMPLE 34

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| L-glutathione | 0.01 |
| Dextran (40.000 - 2.000.000) | 0.05 |
| Sodium chloride | ---- |
| Vitamin E acetate | 0.01 |
| Phosphate buffer | --- |
| Water | up to 100 g |

### EXAMPLE 35

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Hyaluronic acid sodium salt | 0.10 |
| L-glutathione | 0.01 |
| Dextran (40.000 - 2.000.000) | 0.05 |
| Sodium chloride | ---- |
| Vitamin E acetate | 0.01 |
| Phosphate buffer | --- |
| Water | up to 100 g |

### EXAMPLE 36

| **COMPONENT** | **% w/v** |
|---|---|
| L-isoleucine | 0.12 |
| L-leucine | 0.16 |
| L-lysine HCL | 0.18 |
| L-proline | 0.20 |
| L-valine | 0.12 |
| L-glycine | 0.20 |
| L-serine | 0.20 |
| L-alanine | 0.24 |
| Cysteine SH | 0.05 |
| Methionine SH | 0.05 |
| Lipoic acid | 0.10 |
| Magnesium ascorbyl phosphate | 0.20 |
| EDTA | 0.05 |
| Glutamine sulphate | 0.08 |
| L-glutathione | 0.01 |
| Dextran (40.000 - 2.000.000) | 0.05 |
| Sodium chloride | ---- |
| Vitamin E acetate | 0.01 |
| Phosphate buffer | --- |
| Water | up to 100 g |

The solutions of the examples from 1 to 3, from 10 to 14, from 25 to 28 have a value of pH set between 7 and 7,5 and a value of osmolar concentration larger than 480 mOsm/Kg. Instead, the solutions of the examples from 4 to 9, from 15 to 24 and from 29 to 36 have a value of pH set between 5 and 7 and a value of osmolar concentration larger than 480 mOsm/Kg.

## Claims

1. Injectable dermatological composition for treatment of skin wrinkles comprising:
- at least one aminoacid adapted to promote the synthesis of collagen
**characterised in that it provides** furthermore:
- at least one sulphurated aminoacid.

2. Injectable dermatological composition for treatment of skin wrinkles, according to claim 1, wherein said sulphurated aminoacid is selected from the group comprised of:
- cysteine SH;
- methionine SH;
- glutathione,
or a combination thereof.

3. Injectable dermatological composition for treatment of skin wrinkles, according to claim 1, comprising a metalloproteinase inhibitor.

4. Injectable dermatological composition for treatment of skin wrinkles, according to claim 1, wherein said metalloproteinase inhibitor is selected from the group comprised of:
- lipoic acid;
- EDTA;
- EDTA salts;
or a combination thereof.

5. Injectable dermatological composition for treatment of skin wrinkles, according to claim 1, comprising at least one collagen stimulator.

6. Injectable dermatological composition for treatment of skin wrinkles, according to claim 1, comprising at least one natural polymer, in particular, hyaluronic acid.

7. Injectable dermatological composition for treatment of skin wrinkles, according to claim 1, comprising at least one collagen stimulator, in particular, magnesium ascorbyl phosphate.

8. Injectable dermatological composition for treatment of skin wrinkles, according to claim 1, wherein said aminoacids are selected from the group comprised of:
- L-isoleucine;
- L-leucine;
- L-lysine HCL;
- L-proline;
- L-valine;
- L-glycine;
- L-serine;
- L-alanine;
or a combination thereof.

9. Injectable dermatological composition for treatment of skin wrinkles, according to claim 1, comprising at least one natural polymer selected from the group comprised of:
- collagen;
- hyaluronic acid;
- Dextran.

10. Injectable dermatological composition for treatment of skin wrinkles, according to claim 1, said fractions of Dextran are added having have a molecular weight set between 1.000 and 2.000.000 Daltons, advantageously set between 40.000 and 2.000.000 Daltons.

11. Injectable dermatological composition for treatment of skin wrinkles, according to claim 1, comprising an agent suitable to assist the synthesis of collagen, in particular, magnesium ascorbyl phosphate.

12. Injectable dermatological composition for treatment of skin wrinkles, according to claim 1, comprising at least one antioxidant selected from the group comprised of:
- glutathione;
- vitamin E;
a combination thereof.

13. Injectable dermatological composition for treatment of skin wrinkles **characterised in that** it comprises:
- L-isoleucine;
- L-leucine;
- L-lysine HCL;
- L-proline;
- L-valine;
- L-glycine;
- L-serine;
- L-alanine;
- Cysteine SH;
- glutathione GSH;
- Methionine SH.

14. Injectable dermatological composition for treatment of skin wrinkles **characterised in that** it comprises:
- L-isoleucine;
- L-leucine;
- L-lysine HCL;
- L-proline;
- L-valine;
- L-glycine;
- L-serine;
- L-alanine;
- Cysteine SH;
- Methionine SH;
- magnesium ascorbyl phosphate;
- Lipoic acid;
- EDTA;
- EDTA salts.

15. Injectable dermatological composition for treatment of skin wrinkles **characterised in that** it comprises:
- L-isoleucine;
- L-leucine;
- L-lysine HCL;
- L-proline;
- L-valine;
- L-glycine;
- L-serine;
- L-alanine;
- Cysteine SH;
- Methionine SH;
- magnesium ascorbyl phosphate;
- Lipoic acid;
- EDTA (ethylenediaminetetraacetate);
- EDTA salts;
- Hyaluronic acid;
- Dextran.
